# EUROPEAN PATENT APPLICATION

(11) **EP 4 752 153 A1**
(43) Date of publication of application: **03.06.2026**
(21) Application number: 24854453.8
(22) Date of filing: 13.08.2024
(51) Int. Cl.: C07K 7/06, A61K 8/64, A61Q 19/00, A61Q 19/08, A23L 33/18

(54) **NOVEL PEPTIDE HAVING ACTIVITY OF IMPROVING SKIN CONDITION AND USES THEREOF**

(30) Priority: 14.08.2023 KR 20230106435
(71) Applicant: Caregen Co., Ltd., Seoul 06188 (KR)
(72) Inventor: CHUNG, Yong Ji, Seoul 06188 (KR); KIM, Eun Mi, Seoul 06188 (KR); LEE, Eung Ji, Seoul 06188 (KR); KIM, Min Woong, Seoul 06188 (KR); KANG, Hana, Seoul 06188 (KR); HWANG, Bobyeol, Seoul 06188 (KR)
(74) Representative: Bandpay & Greuter
(86) International application number: PCT/KR2024/012055
(87) International publication number: WO 2025/037902

(57) **Abstract**

The present invention relates to a peptide having an activity of improving skin conditions. The peptide of the present invention increases the expression levels of extracellular matrix components and skin barrier function enhancement factors, reduces the expression of skin cell damage factors increased by UV radiation, and restores the expression levels of extracellular matrix components and skin barrier function enhancement factors reduced by UV radiation or heat-shock.

## Description

### [Technical Field]

### [CROSS-REFERENCE TO RELATED APPLICATION]

This application claims the benefit of priority based on Korean Patent Application No. 10-2023-0106435 filed on August 14, 2023, the entire contents of which are incorporated herein as part of this specification.

### [FIELD OF INVENTION]

The present invention relates to a novel peptide having an activity of improving skin conditions and uses thereof.

### [Background Art]

The skin, an organ that covers all of the body's organs and systems, protects the body from external invasion and plays a crucial role in regulating homeostasis by conserving moisture and temperature within the body. Histologically, the skin is composed of three layers: the epidermis, dermis, and subcutis. Skin aging can be divided into intrinsic and extrinsic factors. Intrinsic aging occurs naturally with age and is thought to be caused by a decline in skin cell activity due to genetic factors, such as telomere shortening. Extrinsic aging is caused by environmental factors such as sunlight, cold, wind, and fine dust, of which ultraviolet (UV) radiation and infrared radiation from sunlight are known to be the most important causes.

As skin ages, collagen and elastic fibers, which are fibrous tissues that support the skin's structure, harden. Meanwhile, the ground substance, which fills the spaces between cells and between fibers like collagen and elastic fibers, possesses a remarkably strong ability to retain moisture. As this ground substance depletes, skin elasticity diminishes and wrinkles develop. Representative substances among these ground substances are hyaluronic acid and mucopolysaccharides, and as the skin ages, hyaluronidase increases and the amount of hyaluronic acid in the dermis gradually decreases. In addition, free radicals induced by UV radiation or generated by intracellular respiration react with unsaturated fatty acids in the cell membrane to form a new radical, a peroxyradical, which causes a chain reaction in the presence of oxygen, further inducing lipid peroxidation. Peroxidized lipids can further amplify lipid peroxidation reactions and react with proteins, impairing the function of enzymes or receptor systems. Thus, free radicals are a major cause of damage to skin cells and tissues. In addition, free radicals destroy the skin's antioxidant defense network, which consists of antioxidant enzymes and non-enzymatic antioxidants, thereby tilting the balance between oxidants and antioxidants toward an oxidative state, and continued oxidative stress damages biological components through processes such as lipid peroxidation, protein oxidation, activation of proteolytic enzymes that destroy interstitial components, chain scission and abnormal cross-linking of elastic fibers such as collagen and elastin, cleavage of hyaluronic acid chains, promotion of melanin production, and DNA oxidation.

Hemidesmosomes are the junctions that attach epithelial tissue to the underlying basal layer. Hemidesmosomes are mediated by hemidesmosomal integrin proteins, which bind to keratin filaments on the inner surface while simultaneously binding to laminin in the basal layer on the outer surface of the cell, thereby attaching the cell to the basal layer. In skin structure, hemidesmosomes occur at the dermoepidermal junction, where keratinocytes in the basal layer of the epidermis attach to the extracellular matrix of the dermis. Through this structure, the skin barrier maintains its density firmly.

Patent documents on peptide active substances with skin aging alleviating or skin wrinkle reduction activity are as follows: Korean Patent No. 10-2486996, Korean Patent No. 10-2507392, and International Patent Application No. PCT-KR2021-011278.

### [Prior Art Documents]

### [Patent Documents]

Korean Patent No. 10-2486996,
Korean Patent No. 10-2507392,
International Patent Application No. PCT-KR2021-011278

### [Disclosure]

### [Technical Problem]

The inventors of the present invention have conducted research to develop an effective active substance that can be used to improve skin conditions, including inhibiting skin aging and reducing skin wrinkles. As a result, the inventors experimentally confirmed that the novel peptide they synthesized exhibits excellent activity of improving skin conditions, preventing skin aging and skin wrinkles caused by both intrinsic and extrinsic factors, thereby completing the present invention.

Therefore, it is an object of the present invention to provide a novel peptide having an activity of improving skin conditions.

It is another object of the present invention to provide a composition for improving skin condition comprising the peptide as an active ingredient.

### [Technical Solution]

To achieve the aforementioned objects of the present invention,
one aspect of the present invention provides a peptide comprising an amino acid sequence set forth in SEQ ID NO: 1.

Another aspect of the present invention provides a composition for improving skin conditions comprising the peptide as an active ingredient.

The present invention is described in detail below.

### Peptide and its activity

According to one aspect of the present invention, a peptide comprising an amino acid sequence disclosed in SEQ ID NO: 1 is provided.
[Amino acid sequence of SEQ ID NO: 1]
EIVRKKPI

The term "peptide" as used herein means a linear molecule formed by amino acid residues being linked to each other by peptide bonds.

The peptide comprising the amino acid sequence of SEQ ID NO: 1 of the present invention may be used without modification, but a variant or fragment of amino acids having a different sequence by deletion, insertion, substitution, or a combination thereof of amino acid residues may be used within a range that does not affect the original activity of the peptide, for example, the activity of improving skin condition.

The peptide of the present invention can be modified by phosphorylation, sulfation, acrylation, glycosylation, methylation, farnesylation, etc., within a range that does not change its activity.

The peptide of the present invention includes a peptide comprising an amino acid sequence substantially identical to a peptide comprising the amino acid sequence of SEQ ID NO: 1, and a variant or active fragment thereof. The substantially identical amino acid sequence refers to an amino acid sequence having a sequence identity of at least 75%, for example, at least 80%, at least 85%, at least 90%, at least 95%, at least 97%, or at least 98%, with the amino acid sequence of SEQ ID NO: 1. In addition, the peptide may additionally include a targeting sequence, a tag, a labeled residue, or an amino acid sequence manufactured for a specific purpose to increase half-life or peptide stability.

The peptide of the present invention may be modified at the N-terminus and/or C-terminus to select a portion of the amino acid sequence and enhance its activity. Such N-terminus and/or C-terminus modifications can significantly enhance the stability of the peptide of the present invention, and, for example, increase the half-life of the peptide when administered in vivo. The term "stability" above is meant to include not only stability in vivo, which protects the peptide of the present invention from attack by in vivo protein cleavage enzymes, but also storage stability (e.g., room temperature storage stability).

The N-terminus modification may be one in which a protecting group, selected from the group consisting of an acetyl group, a fluorenylmethoxycarbonyl group, a formyl group, a palmitoyl group, a myristyl group, a stearyl group, and polyethylene glycol (PEG), is attached to the N-terminus of the peptide. The C-terminal modification may be, but is not limited to, one in which a hydroxyl group (-OH), an amino group (-NH2), an azide (-NHNH2), etc. is attached to the C-terminus of the peptide.

The peptide of the present invention can be prepared by various methods widely known in the art to which the present invention pertains. For example, the peptide of the present invention can be prepared by chemical synthesis methods known in the art, particularly solid-phase synthesis techniques (solid-phase synthesis techniques; Merrifield, J. Amer. Chem. Soc. 85:2149-54(1963); Stewart, et al., Solid Phase Peptide Synthesis, 2nd. ed., Pierce Chem. Co.: Rockford, 111(1984)) or liquid-phase synthesis techniques (US Patent No. 5,516,891).

The peptide of the present invention has the activity of improving skin conditions.

In one embodiment, the peptide of the present invention has the following activities with respect to improving skin conditions:
(i) activity that increases the expression levels of genes for extracellular matrix components, such as collagen Iα1, fibronectin, and elastin;
(ii) activity that increases the expression of extracellular matrix components, such as pro-collagen Iα1 and hyaluronic acid;
(iii) activity that increases the expression levels of SIRT1 (Sirtuin 1) gene and AQP3 (Aquaporin 3) gene, which are skin barrier function enhancement factors;
(iv) activity that reduces the level of reactive oxygen species increased by ultraviolet (UV) radiation;
(v) activity that increases the expression levels of genes for extracellular matrix components, such as collagen Iα1, fibronectin, and elastin, reduced by UV radiation;
(vi) activity that increases the expression levels of skin barrier function enhancement factors SIRT1, AQP3, COL17A1, ITGB1, ITGB4, ITGA6, and Plectin reduced by UV radiation; and
(vii) activity that reduces the expression level of MMP-1 increased by UV radiation and heat-shock.

### Composition for improving skin conditions

According to another aspect of the present invention, a composition for improving skin conditions is provided, comprising a peptide comprising an amino acid sequence disclosed in SEQ ID NO: 1 as an active ingredient.

The peptide of the present invention, having the above-described activity, exhibits excellent efficacy in improving skin conditions and can be used for improving skin condition.

In one embodiment, the skin condition improvement in the composition for improving skin conditions may be improvement in skin aging, promotion of skin regeneration, improvement in skin elasticity, reduction in skin wrinkles, or improvement in the skin barrier function.

In one embodiment, the composition for improving skin conditions of the present invention possesses one or more of the following activities:
(i) activity that increases the expression levels of genes for extracellular matrix components, such as collagen Iα1, fibronectin, and elastin;
(ii) activity that increases the expression of extracellular matrix components, such as pro-collagen Iα1 and hyaluronic acid;
(iii) activity that increases the expression levels of the SIRT1 (Sirtuin 1) gene and the AQP3 (Aquaporin 3) gene, which are skin barrier function enhancement factors;
(iv) activity that reduces the level of reactive oxygen species increased by ultraviolet (UV) radiation;
(v) activity that increases the expression levels of genes for extracellular matrix components, such as collagen Iα1, fibronectin, and elastin reduced by UV radiation;
(vi) activity that increases the expression levels of skin barrier function enhancement factors SIRT1, AQP3, COL17A1, ITGB1, ITGB4, ITGA6, and Plectin reduced by UV radiation; and
(vii) activity that reduces the expression level of MMP-1 increased by UV radiation and heat-shock.

The composition for improving skin conditions of the present invention may include the peptide of the present invention at a concentration of 0.01 µM to 1000 µM, and specifically, the peptide of the present invention may be included at a concentration of 0.01 µM to 1000 µM; 0.05 µM to 800 µM, 0.05 µM to 700 µM, 0.05 µM to 600 µM, 0.05 µM to 500 µM, 0.05 µM to 300 µM, 0.05 µM to 200 µM; 0.1 µM to 800 µM, 0.1 µM to 700 µM, 0.1 µM to 600 µM, 0.1 µM to 500 µM, 0.1 µM to 300 µM, 0.1 µM to 200 µM; 1 µM to 800 µM, 1 µM to 700 µM, 1 µM to 600 µM, 1 µM to 500 µM, 1 µM to 300 µM, 1 µM to 200 µM; 5 µM to 800 µM, 5 µM to 700 µM, 5 µM to 600 µM, 5 µM to 500 µM, 5 µM to 300 µM, or 5 µM to 200 µM, but is not limited thereto.

In one embodiment, the composition for improving skin conditions described above may be a cosmetic composition.

The cosmetic composition may be prepared in any formulation commonly manufactured in the technical field to which the present invention pertains, and may be a topical skin preparation. For example, it may be formulated as a solution, suspension, emulsion, paste, gel, cream, lotion, powder, soap, surfactant-containing cleanser, oil, powder foundation, emulsion foundation, wax foundation, and spray, but is not limited thereto.

The cosmetic composition may be prepared in various forms such as a solution, sol-gel, emulsion, oil, wax, aerosol, etc., such as a flexible toner, a nourishing toner, a nourishing cream, a massage cream, an essence, an eye cream, a cleansing cream, a cleansing foam, a cleansing water, a pack, a spray, a powder, a hair tonic, a hair cream, a hair lotion, a hair shampoo, a hair rinse, a hair conditioner, a hair spray, a hair aerosol, a pomade, a gel, etc., but is not limited thereto.

The cosmetic composition of the present invention may include other additives such as excipients and carriers, and it is possible to apply and mix as much as necessary the usual ingredients mixed in general skin cosmetics.

When the formulation of the cosmetic composition is in the form of a paste, cream or gel, as a carrier component, animal oil, vegetable oil, wax, paraffin, starch, tragacanth, cellulose derivatives, polyethylene glycol, silicone, bentonite, silica, talc or zinc oxide may be used.

When the formulation of the cosmetic composition is in the form of a powder or spray, as a carrier component, lactose, talc, silica, aluminum hydroxide, calcium silicate or polyamide powder may be used. In particular, in the case of a spray, a propellant such as chlorofluorohydrocarbon, propane/butane or dimethyl ether may be additionally included, but is not limited thereto.

When the formulation of the cosmetic composition is in the form of a solution or emulsion, a solvent, a solubilizer or an emulsifier may be used as a carrier component, and examples thereof include water, ethanol, isopropanol, ethyl carbonate, ethyl acetate, benzyl alcohol, benzyl benzoate, propylene glycol, 1,3-butyl glycol oil, glycerol aliphatic ester, polyethylene glycol or fatty acid ester of sorbitan.

When the formulation of the cosmetic composition is in the form of a suspension, a liquid diluent such as water, ethanol or propylene glycol, a suspending agent such as ethoxylated isostearyl alcohol, polyoxyethyl sorbitol ester and polyoxyethylene sorbitan ester, microcrystalline cellulose, aluminum metahydroxide, bentonite, agar or tragacanth, etc. can be used as a carrier component.

When the formulation of the cosmetic composition is a surfactant-containing cleansing, aliphatic alcohol sulfate, aliphatic alcohol ether sulfate, sulfosuccinic acid monoester, isethionate, imidazolinium derivative, methyl taurate, sarcosinate, fatty acid amide ether sulfate, alkylamidobetaine, fatty alcohol, fatty acid glyceride, fatty acid diethanolamide, vegetable oil, lanolin derivative, or ethoxylated glycerol fatty acid ester may be used as a carrier component.

When the formulation of the cosmetic composition is a hair shampoo, base ingredients for forming a shampoo, such as a thickener, a surfactant, a viscosity modifier, a moisturizer, a pH adjuster, a preservative, and essential oils, may be mixed. CDE may be used as the thickener; LES, an anionic surfactant, and coco betaine, an amphoteric surfactant, may be used as the surfactant; polyquaternary may be used as the viscosity modifier; glycerin may be used as the moisturizer; and citric acid or sodium hydroxide may be used as the pH adjuster. As the preservative, grapefruit extract, etc. can be used, and in addition, essential oils such as cedarwood, peppermint, and rosemary, silk amino acids, pentanol, or vitamin E can be added.

The ingredients included in the cosmetic composition may further include, in addition to the peptide of the present invention as an active ingredient and the carrier component, ingredients commonly used in cosmetic compositions, such as conventional auxiliary agents such as antioxidants, stabilizers, solubilizers, vitamins, pigments, and fragrances, but are not limited thereto.

According to another aspect of the present invention, a functional food composition for improving skin conditions is provided, which comprises a peptide comprising an amino acid sequence disclosed in SEQ ID NO: 1 as an active ingredient.

The skin condition improvement may be improvement of skin aging, promotion of skin regeneration, improvement of skin elasticity, reduction of skin wrinkles, or improvement of the skin barrier function.

The activity related to improving skin condition in the functional food composition of the present invention is the same as that described in the composition for improving skin condition described above, and therefore, this is cited and will not be repeatedly described.

In the functional food composition of the present invention, the active ingredient, peptide, may be included in an appropriate amount selected within the range of 10 wt% or less, specifically 0.000001 wt% to 10 wt%, based on the total composition weight.

The functional food composition of the present invention may include a food-related effective amount of a peptide and a food-related acceptable carrier.

The functional food composition of the present invention includes not only the peptide as the active ingredient but also ingredients commonly added during food manufacturing, and may include, for example, proteins, carbohydrates, fats, nutrients, seasonings, and flavoring agents. Examples of the carbohydrates mentioned above include common sugars, for example, monosaccharides such as glucose, fructose, etc.; disaccharides such as maltose, sucrose, oligosaccharides, etc.; and polysaccharides such as dextrin, cyclodextrin, etc., and sugar alcohols such as xylitol, sorbitol, erythritol, etc. As flavoring agents, natural flavoring agents, thaumatin, stevia extracts (e.g., rebaudioside A, glycyrrhizin, etc.) and synthetic flavoring agents (saccharin, aspartame, etc.) can be used.

In addition to the above-described ingredients, the functional food composition of the present invention may contain various nutrients, vitamins, minerals (electrolytes), flavoring agents such as synthetic flavoring agents and natural flavoring agents, coloring agents and thickening agents (cheese, chocolate, etc.), pectic acid and its salts, alginic acid and its salts, organic acids, protective colloid thickeners, pH adjusters, stabilizers, preservatives, glycerin, alcohol, carbonating agents used in carbonated beverages, etc. In addition, it may contain fruit pulp for the production of natural fruit juices, fruit juice drinks, and vegetable drinks. For example, when the functional food composition of the present invention is produced as a drink, in addition to the peptide, which is an active ingredient of the present invention, citric acid, high fructose corn syrup, sugar, glucose, acetic acid, malic acid, fruit juice, Eucommia ulmoides extract, jujube extract, licorice extract, etc. may be additionally contained.

There are no specific restrictions on the types of the functional foods. Examples of the functional foods or foods include meat, sausages, bread, chocolate, candy, snacks, confectionery, pizza, ramen, other noodles, gum, dairy products including ice cream, various soups, beverages, tea drinks, alcoholic beverages, vitamin complexes, dairy products, fermented milk, etc., and all functional foods or foods in the conventional sense can be included.

According to another aspect of the present invention, there is provided a pharmaceutical composition for preventing or treating skin aging, comprising a peptide comprising an amino acid sequence disclosed in SEQ ID NO: 1 as an active ingredient.

In one embodiment, the skin aging may be photo-aging of the skin.

In one embodiment, the photo-aging of the skin may be photo-aging caused by UV radiation.

In one embodiment, the photo-aging of the skin may be photo-aging caused by infrared radiation.

In one embodiment, the pharmaceutical composition for preventing or treating skin aging of the present invention or the peptide comprising the amino acid sequence of SEQ ID NO: 1 included in the composition has one or more of the following activities:
(i) activity that increases the expression levels of genes for extracellular matrix components, such as collagen Iα1, fibronectin, and elastin;
(ii) activity that increases the expression of extracellular matrix components, such as pro-collagen Iα1 and hyaluronic acid;
(iii) activity that increases the expression levels of the SIRT1 (Sirtuin 1) gene and the AQP3 (Aquaporin 3) gene, which are skin barrier function enhancement factors;
(iv) activity that reduces the level of reactive oxygen species increased by ultraviolet (UV) radiation;
(v) activity that increases the expression levels of genes for extracellular matrix components, such as collagen Iα1, fibronectin, and elastin reduced by UV radiation;
(vi) activity that increases the expression levels of skin barrier function enhancement factors SIRT1, AQP3, COL17A1, ITGB1, ITGB4, ITGA6, and Plectin reduced by UV radiation; and
(vii) activity that reduces the expression level of MMP-1 increased by UV radiation and heat-shock (infrared radiation).

The pharmaceutical composition of the present invention may comprise a therapeutically effective amount of a peptide comprising the amino acid of SEQ ID NO: 1 of the present invention.

The term "therapeutically effective amount" above means an amount sufficient for the peptide, which is an active ingredient of the pharmaceutical composition of the present invention, to achieve its activity or efficacy, for example, an amount sufficient to achieve the efficacy of treating or preventing skin aging described above.

The term "prevention" as used herein means reducing the risk of developing a disease or disorder, and means any action that inhibits or delays the onset of a disease by preventing the disease or one or more of its clinical symptoms from progressing.

The term "treatment" as used herein means alleviating a disease or disorder, and includes any action that improves or beneficially alters the symptoms of a disease by arresting or reducing the progression of the disease or one or more of its clinical symptoms.

The pharmaceutical composition of the present invention may include a pharmaceutically acceptable carrier.

The pharmaceutically acceptable carrier is one commonly used in formulations, and includes, but is not limited to, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate, and mineral oil.

The pharmaceutical composition of the present invention may additionally include, in addition to the above ingredients, a lubricant, a wetting agent, a sweetener, a flavoring agent, an emulsifier, a suspending agent, a preservative, etc., but is not limited thereto.

Suitable pharmaceutically acceptable carriers and formulations are described in detail in Remington: The Science and Practice of Pharmacy, (21st ed., 2005, Lippincott Williams & Wilkins).

The pharmaceutical composition of the present invention can be administered by any suitable route for treating skin aging, for example, it can be administered orally or parenterally, and in the case of parenteral administration, it can be administered by intravenous injection, subcutaneous injection, intramuscular injection, intraperitoneal injection, topical administration, transdermal administration, skin application, etc.

The dosage of the above pharmaceutical composition may be, but is not limited to, 0.0001 µg to 1000 mg, 0.001 µg to 1000 mg, 0.01 ng to 1000 mg, 0.1 µg to 1000 mg, 0.1 µg to 500 mg, or 1.0 µg to 1000 mg per day, and may be prescribed in various ways depending on factors such as formulation method, administration method, patient age, weight, sex, pathological condition, food, administration time, administration route, excretion rate, and reaction sensitivity.

The pharmaceutical composition of the present invention can be prepared in a unit dose form or can be prepared by inserting it into a multi-dose container by formulating it using a pharmaceutically acceptable carrier and/or excipient according to a method that can be easily performed by a person having ordinary skill in the art to which the present invention pertains. In this case, the formulation may be in the form of a solution, suspension or emulsion in an oil or aqueous medium, or in the form of an extract, powder, granules, tablet or capsule, and may additionally include a dispersing agent or stabilizer.

The pharmaceutical composition of the present invention may contain the peptide of the present invention at a concentration of 0.01 µM to 1000 mM, specifically, the peptide of the present invention may contain the peptide of the present invention at a concentration of 0.01 µM to 900 mM, 0.01 µM to 500 mM, 0.01 µM to 300 mM, 0.01 µM to 100 mM, 0.01 µM to 50 mM, 0.01 µM to 30 mM, 0.01 µM to 10 mM, 0.01 µM to 1 mM, 0.01 µM to 0.1 mM, 0.01 µM to 0.01 mM, 1.01 µM to 1000 µM; 0.05 µM to 800 µM, 0.05 µM to 700 µM, 0.05 µM to 600 µM, 0.05 µM to 500 µM, 0.05 µM to 300 µM, 0.05 µM to 200 µM; 0.1 µM to 800 µM, 0.1 µM to 700 µM, 0.1 µM to 600 µM, 0.1 µM to 500 µM, 0.1 µM to 300 µM, 0.1 µM to 200 µM; 1 µM to 800 µM, 1 µM to 700 µM, 1 µM to 600 µM, 1 µM to 500 µM, 1 µM to 300 µM, 1 µM to 200 µM; 5 µM to 800 µM, 5 µM to 700 µM, 5 µM to 600 µM, 5 µM to 500 µM, 5 µM to 300 µM, or 5 µM to 200 µM, but is not limited thereto.

### Use of Peptide

According to another aspect of the present invention, a peptide comprising the amino acid sequence disclosed in SEQ ID NO: 1 is provided for use in improving skin conditions.

According to another aspect of the present invention, a peptide comprising the amino acid sequence disclosed in SEQ ID NO: 1 is provided for use in the manufacture of cosmetics or functional foods for improving skin conditions.

According to another aspect of the present invention, a method for improving a skin conditions is provided, comprising administering to a patient or subject in need of improvement of a skin conditions a peptide comprising an amino acid sequence disclosed in SEQ ID NO: 1 or a composition comprising the peptide as an active ingredient.

The term "patient" or "subject" as used herein refers to a human or a non-human animal, such as a human, a primate, a mammal, and a vertebrate. According to another aspect of the present invention, there is provided a use of a peptide comprising an amino acid sequence disclosed in SEQ ID NO: 1 for treating or preventing skin aging.

According to another aspect of the present invention, there is provided a use of a peptide comprising the amino acid sequence disclosed in SEQ ID NO: 1 for the manufacture of a medicament for treating or preventing skin aging. According to another aspect of the present invention, there is provided a method for treating skin aging, comprising administering to a patient or subject in need of treatment of skin aging a peptide comprising the amino acid sequence disclosed in SEQ ID NO: 1.

The term "patient" or "subject" as used herein refers to a human or non-human animal, such as humans, primates, mammals, and vertebrates.

The above-described uses, methods for improving skin conditions, and methods for treating skin aging are all equally applicable to the peptides of the present invention, their activities, and compositions, and are not described repeatedly to avoid excessive complexity of the specification.

### [Advantageous Effects]

The peptide of the present invention has an activity of improving skin conditions. The peptide of the present invention increases the expression levels of extracellular matrix components and skin barrier function enhancement factors, reduces the expression of skin cell damage factors increased by UV radiation, and restores the expression levels of extracellular matrix components and skin barrier function enhancement factors reduced by UV radiation or heat-shock.

However, the effects of the present invention are not limited to the effects mentioned above, and other effects not mentioned will be clearly understood by those skilled in the art from the description below.

### [Description of Drawings]

FIGS. 1A and 1B show that the peptide of the present invention increases the mRNA expression levels of genes for collagen Iα1 (COLA1), fibronectin (FN1), and elastin (ELN) in NIH3T3 cells.
FIG. 2 shows that the peptide of the present invention increases the secretion of Pro-Collagen Iα1 and hyaluronic acid in NIH3T3 cells and HaCaT cells.
FIG. 3 shows that the peptide of the present invention increases the mRNA expression levels of SIRT1 (Sirtuin 1) gene and AQP3 (Aquaporin 3) gene in HaCaT cells.
FIGS. 4A and 4B show that the peptide of the present invention reduces intracellular reactive oxygen species (ROS) levels increased by UV radiation in NIH3T3 cells and HaCaT cells again.
FIGS. 5A and 5B show that the peptide of the present invention increases the mRNA expression levels of the genes for collagen Iα1 (COLA1), fibronectin (FN1), and elastin (ELN) again, which were reduced by UV radiation in NIH3T3 cells.
FIGS. 6A and 6B show that the peptide of the present invention increases the mRNA levels of SIRT1 and AQP3 again, which were reduced by UV radiation in HaCaT cells.
FIGS. 6C and 6D show that the peptide of the present invention increases the mRNA levels of COL17A1 (Collagen XVIIα1), ITGB1 (Integrin β 1), ITGB4 (Integrin β 4), ITGA6 (Integrin α 6), and Plectin again, which were reduced by UV radiation in HaCaT cells.
FIGS. 7A and 7B show that the peptide of the present invention reduces the expression of MMP-1 increased by UV radiation in NIH3T3 cells and HaCaT cells again.
FIG. 8 shows that the peptide of the present invention reduces the expression of MMP-1 increased by heat-shock in NIH3T3 cells again.

### [Mode for Invention]

Hereinafter, the present invention will be described in detail by way of examples. However, the following examples specifically illustrate the present invention, and the content of the present invention is not limited by the following examples.

### Preparation Example 1: Preparation of peptide

A peptide having the amino acid sequence of SEQ ID NO: 1 shown in Table 1 below was synthesized using an automatic peptide synthesizer (Milligen 9050, Millipore, USA), and the synthesized peptide was purified using C18 reverse-phase high-performance liquid chromatography (HPLC) (Waters Associates, USA). The column used was ACQUITY UPLC BEH300 C18 (2.1 mm X 100 mm, 1.7 µm, Waters Co, USA).

**[Table 1]**

| SEQ ID NO | Amino acid sequence (N-terminus -> C-terminus) |
|---|---|
| 1 | EIVRKKPI |

The efficacy of the peptide of SEQ ID NO: 1 prepared above was evaluated through the following experiments.

### Experimental Example 1: Analysis of gene expression of extracellular matrix components

The effect of the peptides prepared in Preparation Example 1 on the expression of extracellular matrix (ECM) components was evaluated by measuring the mRNA expression levels of the genes of each component.

NIH3T3 cells (mouse fibroblast cell line) were seeded at a density of 3 × 10⁵ cells/well in 6-well plates and cultured for 24 hours. The cultured cells were washed once with serum-free DMEM medium. The peptides prepared in Preparation Example 1 were mixed in serum-free DMEM medium at different concentrations to prepare peptide medium solutions of 10 µM, 50 µM, and 100 µM, which were then added to the cells. Cells were cultured in a CO₂ incubator at 37°C for 24 hours, washed twice with PBS, and RNA was isolated using easy blue (iNtRON, Cat. No.: 17061, Korea). The amount of RNA was quantified, 1000 ng of RNA per tube was added, and cDNA synthesis was performed using an RT kit (enzynomics, Cat. No.: RT200, Korea), followed by PCR performed using a PCR kit (enzynomics, Cat. No.: P581T, Korea). Primers used for PCR are shown in Table 2 below.

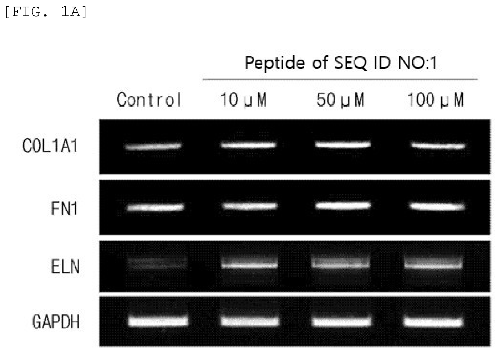

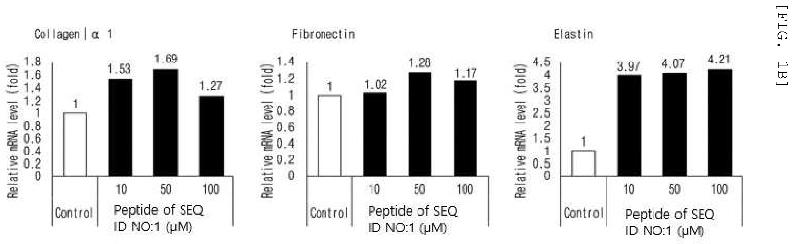

As a result of the experiment, it was confirmed that the mRNA expression level of the genes of collagen Iα1 (Collagen, type I, alpha 1), fibronectin, and elastin, which are components of the extracellular matrix (ECM), increased by the peptide of SEQ ID NO: 1 in NIH3T3 cells (FIG. 1).

### Experimental Example 2: Analysis of protein expression of extracellular matrix components

The effect of the peptide prepared in Preparation Example 1 on the expression of extracellular matrix (ECM) components was evaluated by measuring the expression levels of Collagen Iα1 and HA (Hyaluronic acid).

NIH3T3 cells (mouse fibroblast cell line) and HaCaT cells (human keratinocyte cell line) were seeded at a density of 1 x 10⁴ cells/well in a 24-well plate and cultured for 24 hours. The cultured cells were washed once with serum-free DMEM medium. The peptides prepared in Preparation Example 1 were mixed in serum-free DMEM medium at different concentrations to prepare peptide medium solutions of 10 µM, 50 µM, and 100 µM, which were then added to the cells. Cells were cultured in a CO₂ incubator at 37°C for 72 hours. NIH3T3 and HaCaT cell cultures were centrifuged to settle cells and impurities, and only the supernatant of the culture medium was taken and transferred to an Eppendorf tube. The resulting cultures were used for Pro-Collagen ELISA (abcam, Cat No.: ab210579, UK) and HA (Hyaluronic acid) ELISA (ECHELON biosciences, Cat No.: K-1200, USA).

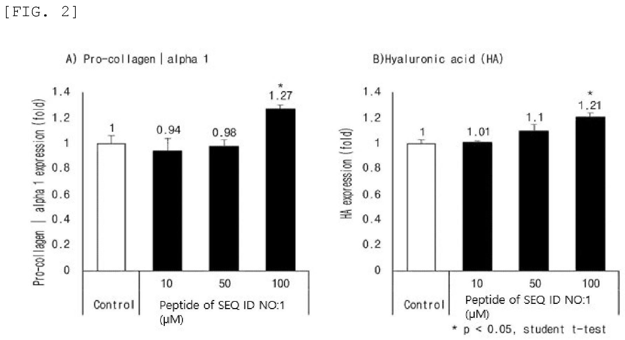

As a result of the experiment, it was confirmed that the secretion of Pro-Collagen Iα1, one of the components constituting the extracellular matrix, was increased by the peptide of SEQ ID NO: 1 in NIH3T3 cells (FIG. 2). In addition, it was confirmed that the secretion of Hyaluronic acid (HA), one of the components constituting the extracellular matrix, was increased by the peptide of SEQ ID NO: 1 in HaCaT cells (FIG. 2).

### Experimental Example 3: Analysis of expression of skin barrier function-related genes

The effect of the peptides prepared in Preparation Example 1 on the expression of genes related to the skin barrier function was evaluated.

HaCaT cells (human keratinocyte cell line) were seeded at a density of 3 x 10⁵ cells/well in 6-well plates and cultured for 24 hours. The cultured cells were washed once with serum-free DMEM medium. The peptides prepared in Preparation Example 1 were mixed in serum-free DMEM medium at different concentrations to prepare peptide medium solutions of 10 µM, 50 µM, and 100 µM, which were then added to the cells. Cells were cultured in a CO₂ incubator at 37°C for 24 hours, washed twice with PBS, and then RNA was isolated using easy blue (iNtRON, Cat. No.: 17061, Korea). The amount of RNA was quantified, and 1,000 ng of RNA per tube was added, and then cDNA was synthesized using an RT kit (enzynomics, Cat. No.: RT200, Korea), followed by PCR using a PCR kit (enzynomics, Cat. No.: P581T, Korea). Primers used for PCR are shown in Table 2.

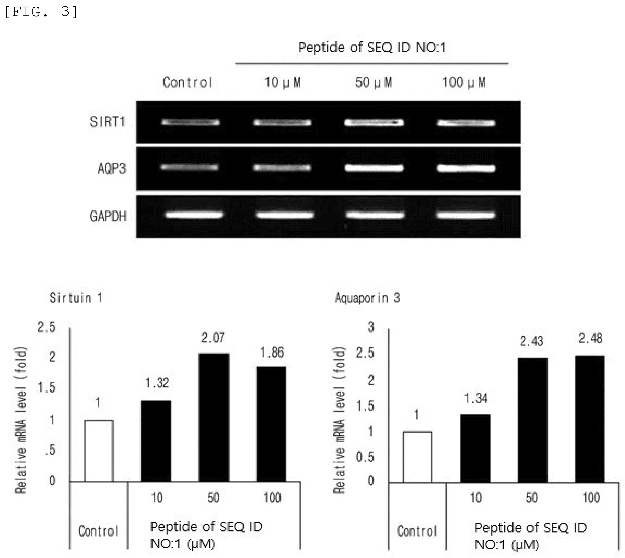

SIRT1 (Sirtuin 1) and AQP3 (Aquaporin 3) are known to be factors that affect the maintenance of the skin barrier function by regulating the aging and moisturizing of cells constituting the epidermis. Experimental results confirmed that the peptide of SEQ ID NO: 1 increased the mRNA expression levels of the SIRT1 (Sirtuin 1) gene and AQP3 (Aquaporin 3) gene, which are skin barrier function enhancement factors, in HaCaT cells (FIG. 3).

### Experimental Example 4: Inhibition of UV-induced reactive oxygen species (ROS) production

It was evaluated whether the peptide prepared in Preparation Example 1 inhibits the production of reactive oxygen species (ROS) induced by UV radiation.

NIH3T3 cells (mouse fibroblast cell line) and HaCaT cells (human keratinocyte cell line) were seeded at a density of 5 x 10⁵ cells/well in 6-well plates and cultured for 24 hours. The cultured cells were washed once with serum-free DMEM medium. The peptides prepared in Preparation Example 1 were mixed in serum-free DMEM medium at various concentrations to prepare peptide medium solutions of 10 µM, 50 µM, and 100 µM, which were then added to the cells. The cells to which the peptides were added were cultured in a CO₂ incubator at 37°C for 1 hour. After culture, the medium was removed from the cells, and PBS was added. Using a UV radiator (VILBER LOURMAT, Cat No.: 3102-BSU, France), NIH3T3 cells were irradiated with UVA at 6 J/cm², and HaCaT cells were irradiated with UVB at 15 mJ/cm². After removing PBS, the peptide medium solutions at each concentration mentioned above were added to the cells again. The cells added with the peptides were cultured in a CO₂ incubator at 37°C for 24 hours. After treatment with 10 µM DCFH-DA (2', 7'-dichlorofluorescein diacetate), the cells were wrapped in foil and cultured in a CO₂ incubator at 37°C for 30 minutes. After washing twice with PBS, the cells were harvested and the fluorescence values were measured by FACS.

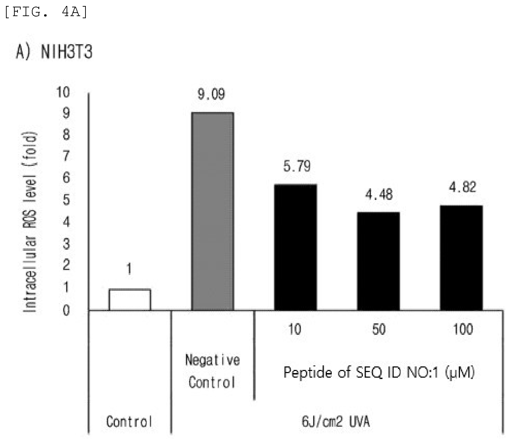

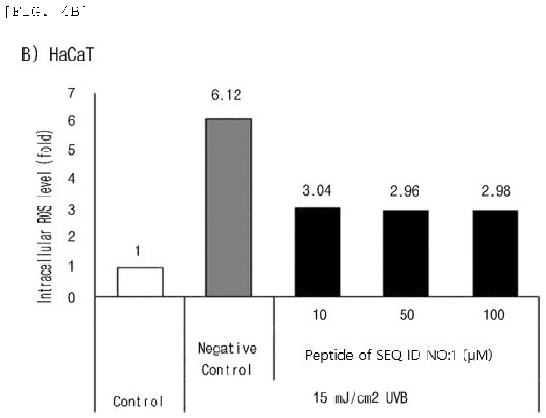

Experimental results confirmed that the peptide of SEQ ID NO: 1 reduced the intracellular ROS level increased by UV radiation in NIH3T3 cells (FIG. 4A). In addition, it was confirmed that the peptide of SEQ ID NO: 1 again reduced the intracellular ROS level increased by UV radiation in HaCaT cells (FIG. 4B).

### Experimental Example 5: Restoration of extracellular matrix gene expression reduced by UV radiation

It was evaluated whether the peptide of SEQ ID NO: 1 prepared in Preparation Example 1 restores the expression levels of extracellular matrix genes reduced by UV radiation.

NIH3T3 cells (mouse fibroblast cell line) were seeded at a density of 5 x 10⁵ cells/well in 6-well plates and cultured for 24 hours. The cultured cells were washed once with serum-free DMEM medium. The peptides prepared in Preparation Example 1 were mixed in serum-free DMEM medium at various concentrations to prepare peptide medium solutions of 10 µM, 50 µM, and 100 µM, which were then added to the cells. The cells to which the peptides were added were cultured in a CO₂ incubator at 37°C for 1 hour. After culturing the cells, the medium was removed from the cells and PBS was added. UVA was irradiated at 8 J/cm² using a UV radiator (VILBER LOURMAT, Cat No.: 3102-BSU, France). After removing PBS, the peptide medium solution at each concentration mentioned above was added again to the cells. Peptide-added cells were cultured in a CO₂ incubator at 37°C for 6 hours. After washing twice with PBS, RNA was isolated using easy blue (iNtRON, Cat. No.: 17061, Korea). The amount of RNA was quantified, and 1,000 ng of RNA per tube was added, and then cDNA synthesis was performed using an RT kit (enzynomics, Cat. No.: RT200, Korea). Subsequently, PCR was performed using a PCR kit (enzynomics, Cat. No.: P581T, Korea). Primers used for PCR are shown in Table 2.

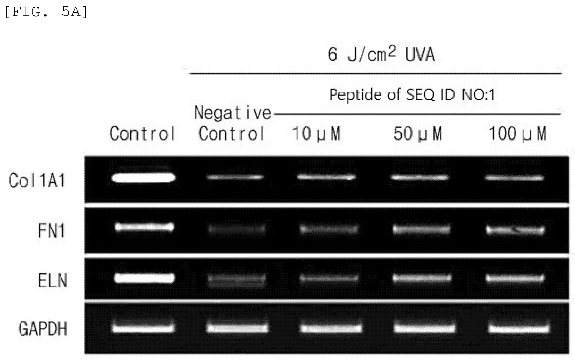

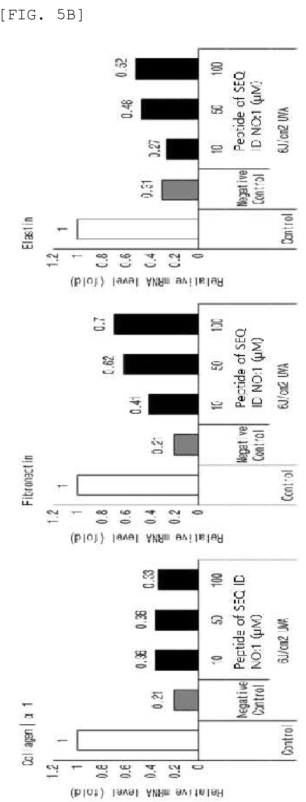

Experimental results showed that the mRNA expression levels of collagen Iα1, fibronectin, and elastin genes were reduced by UV radiation in NIH3T3 cells. However, when the peptide of SEQ ID NO: 1 was treated, the expression levels of the genes that had been reduced were increased again (FIGS. 5A and 5B).

### Experimental Example 6: Restoration of skin barrier function-related gene expression reduced by UV radiation

**It** was evaluated whether the peptide of SEQ ID NO: 1 prepared in Preparation Example 1 restores the expression level of skin barrier function-related genes reduced by UV radiation.

HaCaT cells (human keratinocyte cell line) were seeded at a density of 5 x 10⁵ cells/well in 6-well plates and cultured for 24 hours. The cultured cells were washed once with serum-free DMEM medium. The peptides prepared in Preparation Example 1 were mixed in serum-free DMEM medium at various concentrations to prepare peptide medium solutions of 10 µM, 50 µM, and 100 µM, which were then added to the cells. The cells to which the peptides were added were cultured in a CO₂ incubator at 37°C for 1 hour. After culturing the cells, the medium was removed from the cells and PBS was added. UVB was irradiated at 20 mJ/cm² using a UV irradiator (VILBER LOURMAT, Cat. No.: 3102-BSU, France). After removing the PBS, the peptide medium solution at each concentration mentioned above was added to the cells again. The cells with the peptides added were cultured in a CO₂ incubator at 37°C for 4 hours. After washing twice with PBS, RNA was isolated using easy blue (iNtRON, Cat. No.: 17061, Korea). The amount of RNA was quantified, and 1,000 ng of RNA per tube was added, and cDNA synthesis was performed using an RT kit (enzynomics, Cat. No.: RT200, Korea). Subsequently, PCR was performed using a PCR kit (enzynomics, Cat. No.: P581T, Korea). The primers used for PCR are shown in Table 2.

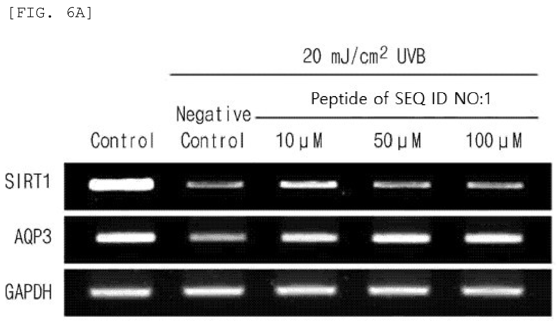

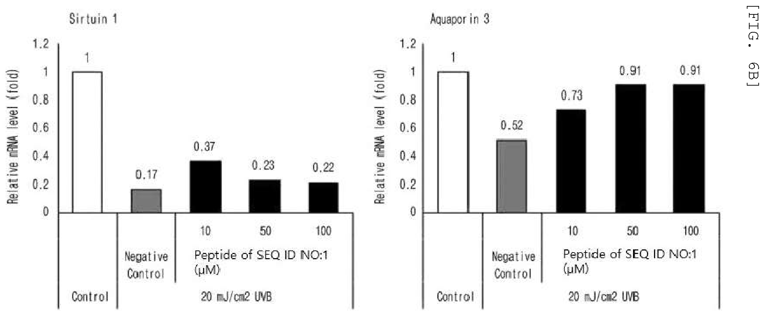

SIRT1 (Sirtuin 1) and AQP3 (Aquaporin 3) are known to be factors that affect the maintenance of the skin barrier function by regulating the aging and moisturizing of cells constituting the epidermis. Experimental results showed that the mRNA levels of SIRT1 and AQP3 were reduced by UV radiation in HaCaT cells, and treatment with the peptide of SEQ ID NO: 1 restored the reduced mRNA expression levels of these genes again (FIGS. 6A and 6B).

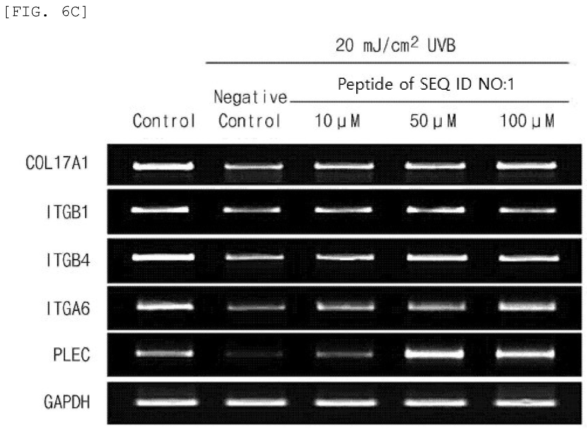

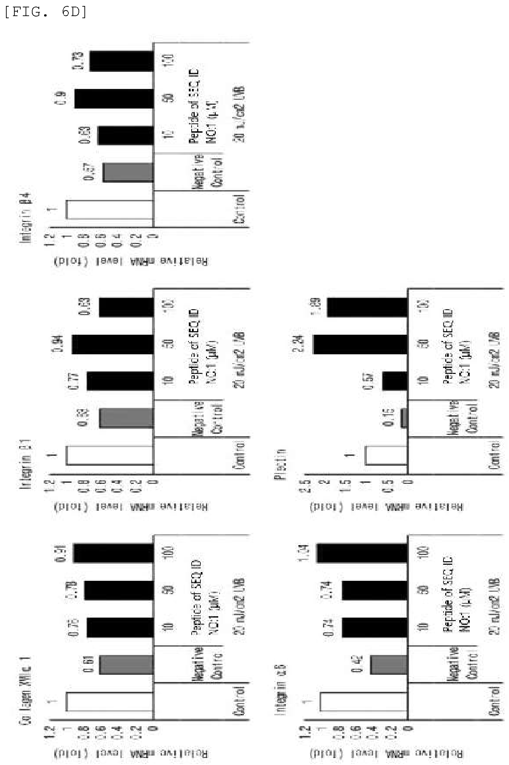

Hemidesmosomes are the region where keratinocytes in the basal layer of the epidermis attach to the extracellular matrix (ECM) of the dermis, thereby maintaining the density of the skin barrier. Experimental results showed that in HaCaT cells, the mRNA levels of hemidesmosome components COL17A1 (Collagen XVIIα1), ITGB1 (Integrin β 1), ITGB4 (Integrin β 4), ITGA6 (Integrin α 6), and Plectin were reduced by UV radiation, and the mRNA expression levels of these genes that had been reduced were restored by treatment with the peptide of SEQ ID NO: 1 (FIG. 6C and FIG. 6D).

### Experimental Example 7: Inhibition of UV-induced MMP-1 expression

The effect of the peptide of SEQ ID NO: 1 prepared in Preparation Example 1 on UV-induced MMP-1 expression was evaluated.

NIH3T3 cells (mouse fibroblast cell line) and HaCaT cells (human keratinocyte cell line) were seeded at a density of 5 x 10⁵ cells/well in 6-well plates and cultured for 24 hours. The cultured cells were washed once with serum-free DMEM medium. The peptides prepared in Preparation Example 1 were mixed in serum-free DMEM medium at various concentrations to prepare peptide medium solutions of 10 µM, 50 µM, and 100 µM, which were added to the cells. The cells to which the peptides were added were cultured in a CO₂ incubator at 37°C for 1 hour. After culturing the cells, the medium was removed from the cells, and PBS was added. NIH3T3 cells were irradiated with UVA at 6 J/cm² and HaCaT cells were irradiated with UVB at 15 mJ/cm² using a UV radiator (VILBER LOURMAT, Cat No.: 3102-BSU, France). After removing PBS, the peptide medium solutions at each concentration were added to the cells. The cells with the peptides added were cultured in a CO₂ incubator at 37°C for 24 hours. After washing twice with PBS, cells were lysed with cell lysis buffer. After processing with 5X sample buffer, SDS-PAGE was performed using a 10% SDS-PAGE gel. Proteins separated by SDS-PAGE were transferred to PVDF membranes. Blocking was performed with 5% skim milk for 1 hour at room temperature. Anti-MMP-1 antibody (Abcam, Cat No.: ab137332, UK) was diluted 1:1000 in 5% skim milk and reacted with the membrane for 2 hours. The loading control was reacted in the same manner using anti-α-tubulin antibody (Santa Cruz Biotechnology, Cat No.: sc69969, USA). The cells were washed three times for 10 minutes each with 0.1% PBS-T (0.1% Tween-20 in PBS). Goat Anti-Rabbit IgG (H+L) secondary antibody (Jackson ImmunoResearch, Cat. No.: 111-035-003, USA) was diluted 1:3000 in 5% skim milk and reacted with the membrane for 1 hour. The secondary antibody against α-tubulin, a loading control, was reacted in the same manner using the same product. After treatment with ECL solution (GE Healthcare, Cat. No.: RPN2232, USA), the expression level was analyzed using ImageQuant 800 (Cytiva, Cat. No.: 29-3994-81, USA) equipment.

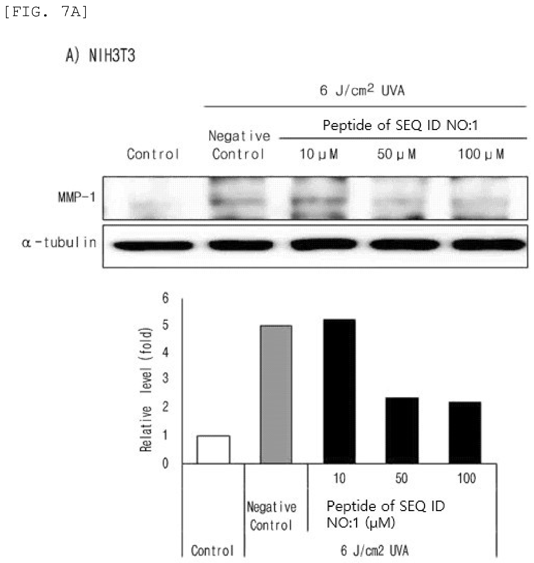

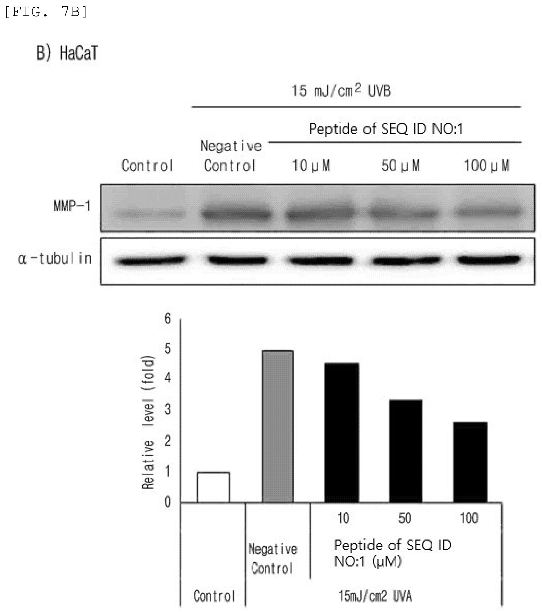

Experimental results showed that UV radiation increased the expression of MMP-1 in NIH3T3 cells, and the peptide of SEQ ID NO: 1 inhibited the UV-radiation-induced increase in MMP-1 expression (FIG. 7A). In addition, UV radiation increased the expression of MMP-1 in HaCaT cells, and the peptide of SEQ ID NO: 1 inhibited the UV-radiation-induced increase in MMP-1 expression (FIG. 7B).

### Experimental Example 8: Inhibition of heat-induced MMP-1 expression

The effect of the peptide of SEQ ID NO: 1 prepared in Preparation Example 1 on heat-induced MMP-1 expression was evaluated.

NIH3T3 cells (mouse fibroblast cell line) were seeded at a density of 5 x 10⁵ cells/well in 6-well plates and cultured for 24 hours. The cultured cells were washed once with serum-free DMEM medium. The peptides prepared in Preparation Example 1 were mixed with serum-free DMEM medium at various concentrations to prepare peptide medium solutions of 10 µM, 50 µM, and 100 µM, which were added to the cells. The cells to which the peptides were added were cultured in a CO₂ incubator at 37°C for 1 hour. The lid and lower plate of the 6-well plate were sealed with paraffin film, and then the lower plate was submerged in a 44°C water bath and incubated for 30 minutes. After removing the existing medium, the medium solution containing the peptide dissolved at the above concentrations was added again, and the plate was incubated in a 37°C CO₂ incubator for 8 hours. After washing the cells twice with PBS, the cells were lysed with cell lysis buffer. After processing with 5X sample buffer, SDS-PAGE was performed using a 10% SDS-PAGE gel. Proteins separated by SDS-PAGE were transferred to PVDF membranes. Blocking was performed with 5% skim milk for 1 hour at room temperature. Anti-MMP-1 antibody (Abcam, Cat No.: ab137332, UK) was diluted 1:1000 in 5% skim milk and reacted with the membrane for 2 hours. The loading control was reacted in the same manner using anti-α-tubulin antibody (Santa Cruz Biotechnology, Cat No.: sc69969, USA). The cells were washed three times for 10 minutes each with 0.1% PBS-T (0.1% Tween-20 in PBS). Goat Anti-Rabbit IgG (H+L) secondary antibody (Jackson ImmunoResearch, Cat. No.: 111-035-003, USA) was diluted 1:3000 in 5% skim milk and reacted with the membrane for 1 hour. The secondary antibody against α-tubulin, which served as a loading control, was reacted in the same manner using the same product. After treatment with ECL solution (GE Healthcare, Cat. No.: RPN2232, USA), the expression level was analyzed using ImageQuant 800 (Cytiva, Cat. No.: 29-3994-81, USA) equipment.

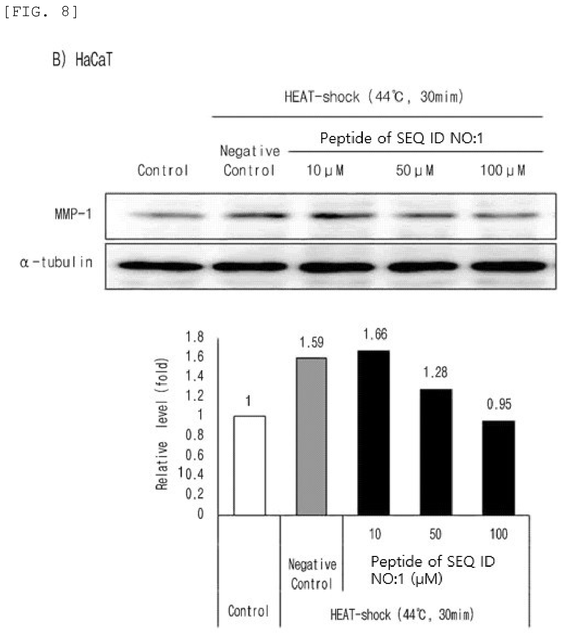

As a result of the experiment, the expression of MMP-1 was increased by heat-shock in NIH3T3 cells, and the peptide of SEQ ID NO: 1 inhibited the increase in MMP-1 expression induced by heat-shock (FIG. 8).

**[Table 2]**

| Primer | Sequence (5' -> 3') | SEQ ID NO |
|---|---|---|
| Collal Forward | (5') CAC CCT CAA GAG CCT GAG TC (3') | 2 |
| Collal Reverse | (5') AGA CGG CTG AGT AGG GAA CA (3') | 3 |
| Fibronectin Forward | (5') CCA GGA ACC GAG TAC ACC AT (3') | 4 |
| Fibronectin Reverse | (5') ATA CCC AGG TTG GGT GAT GA (3') | 5 |
| Elastin Forward | (5') GCAAGACCTGGCTTTGGACT (3') | 6 |
| Elastin Reverse | (5') GGGAGTTTCTGGTTAGGGCTG (3') | 7 |
| AQP3 Forward | (5') CCT TCT TGG GTG CTG GAA TA (3') | 8 |
| AQP3 Reverse | (5') ACA CGA TAA GGG AGG CTG TG (3') | 9 |
| SIRT1 Forward | (5') TCA GTG GCT GGA ACA GTG AG (3') | 10 |
| SIRT1 Reverse | (5') TCT GGC ATG TCC CAC TAT CA (3') | 11 |
| COL17A1 Forward | (5') CTG ACT TTG CTG GAG ATC TGG (3') | 12 |
| COL17A1 Reverse | (5') TAG GCC ATC CCT TGC AGT AG (3') | 13 |
| ITGB1 Forward | (5') CGC CGC GCG GAA AAG ATG (3') | 14 |
| ITGB1 Reverse | (5') AAA CAC CAG CAG CCG TGT AA (3') | 15 |
| ITGB4 Forward | (5') CGA GGT AGG TCC AGG ACG G (3') | 16 |
| ITGB4 Reverse | (5') GGC TCC TTC AGC TTC TCA GG (3') | 17 |
| ITGA6 Forward | (5') AAA AGG GTG TTG GGA GGG TG (3') | 18 |
| ITGA6 Reverse | (5') GAA TCC CAT TGC TTT GGC AC (3') | 19 |
| Plectin Forward | (5') CAC CTC ATC AAG GCC CAG AG (3') | 20 |
| Plectin Reverse | (5') AGC TGG AGG TGA AGT TGT CG (3') | 21 |
| GAPDH Forward | (5') GGA GCC AAA AGG GTC ATC AT (3') | 22 |
| GAPDH Reverse | (5') GTG ATG GCA TGG ACT GTG GT (3') | 23 |

Although representative embodiments of the present application have been described above as examples, the scope of the present application is not limited only to the specific embodiments described above, and a person with ordinary knowledge in the relevant field will be able to make appropriate changes within the scope described in the claims of the present application.

## Claims

1. A peptide comprising an amino acid sequence of SEQ ID NO: 1.

2. A composition for improving skin conditions comprising the peptide of claim 1 as an active ingredient.

3. The composition for improving skin conditions of claim 2, wherein the improvement of skin conditions comprises improvement of skin aging, promotion of skin regeneration, improvement of skin elasticity, reduction of skin wrinkles, or improvement of skin barrier function.

4. The composition for improving skin conditions of claim 2, wherein the composition has activity of improving skin conditions through one or more of the following activities:
(i) activity that increases expression levels of genes for collagen Iα1, fibronectin, and elastin;
(ii) activity that increases the expression of pro-collagen Iα1 and hyaluronic acid;
(iii) activity that increases the expression levels of SIRT1 (Sirtuin 1) gene and AQP3 (Aquaporin 3) gene, which are skin barrier function enhancement factors;
(iv) activity that reduces the level of reactive oxygen species increased by ultraviolet (UV) radiation;
(v) activity that increases the expression levels of collagen Iα 1, fibronectin, and elastin genes reduced by UV radiation;
(vi) activity that increases the expression levels of skin barrier function enhancement factors SIRT1, AQP3, COL17A1, ITGB1, ITGB4, ITGA6, and Plectin reduced by UV radiation; and
(vii) activity that reduces the expression level of MMP-1 increased by UV radiation and heat-shock.

5. The composition for improving skin conditions of claim 2, wherein the composition is a cosmetic composition.

6. The composition for improving skin conditions of claim 5, wherein the cosmetic composition is in a formulation selected from the group consisting of a solution, a suspension, an emulsion, a paste, a gel, a cream, a lotion, a powder, a soap, a surfactant-containing cleanser, an oil, a powder foundation, an emulsion foundation, a wax foundation, and a spray.

7. A functional food composition for improving skin conditions comprising a peptide having an amino acid sequence of SEQ ID NO: 1 as an active ingredient.

8. The functional food composition for improving skin conditions of claim 7, wherein the improvement of skin conditions comprises improvement of skin aging, promotion of skin regeneration, improvement of skin elasticity, reduction of skin wrinkles, or improvement of the skin barrier function.

9. A pharmaceutical composition for preventing or treating skin aging, comprising a peptide having an amino acid sequence of SEQ ID NO: 1 as an active ingredient.

10. The pharmaceutical composition for preventing or treating skin aging of claim 9, wherein the skin aging is photo-aging of the skin.

11. The pharmaceutical composition for preventing or treating skin aging of claim 10, wherein the photo-aging of the skin is photo-aging caused by ultraviolet(UV) radiation.

12. The pharmaceutical composition for preventing or treating skin aging of claim 10, wherein the photo-aging of the skin is photo-aging caused by infrared (IR) radiation.
